# EUROPEAN PATENT APPLICATION

(11) **EP 1 845 405 A1**
(43) Date of publication of application: **17.10.2007**
(21) Application number: 05809155.4
(22) Date of filing: 22.11.2005
(51) Int. Cl.: G02C 13/00, A61K 31/717, A61K 47/34, C11D 1/72, C11D 3/20, G02C 7/04

(54) **LIQUID AGENT COMPOSITION FOR CONTACT LENS**

(30) Priority: 06.12.2004 JP 2004353394
(71) Applicant: Menicon Co., Ltd., Nagoya-shi, Aichi 460-0006 (JP)
(72) Inventor: TANIKAWA, Sadayasu, MENICON CO., LTD., Kasugai-shi, Aichi 4870032 (JP); NOMURA, Hideshi, MENICON CO., LTD., Kasugai-shi, Aichi 4870032 (JP)
(74) Representative: Paget, Hugh Charles Edward
(86) International application number: PCT/JP2005/021438
(87) International publication number: WO 2006/061990

(57) **Abstract**

To provide a solution composition for a contact lens high in safety to living organisms, which has a function of being able to remove stains such as a lipid stain adhered to the contact lens and a function of being able to effectively inhibit adhesion of stains such as a protein to a surface of the contact lens, and does not cause damage to the cornea due to damage of a tear fluid layer. A specific nonionic surfactant is contained in the vicinity of the critical micelle concentration, and polyethylene glycol is contained at a large ratio to such a nonionic surfactant.

## Description

### TECHNICAL FIELD

The present invention relates to a solution composition for a contact lens, and particularly to a solution composition for a contact lens which is enhanced in safety to the eye, while ensuring a function of removing stains on a contact lens or a function of preventing adhesion of stains thereto, and can be suitably used as an eye drop.

Conventionally, contact lenses have been used, being classified into non-water-content contact lenses and water-content contact lenses, or roughly divided into hard contact lenses and soft contact lenses. In all these contact lenses, stains such as proteins or eye lipids derived from tear fluid are liable to be adhered to and deposited on surfaces of the contact lenses in their wearing. Further, stains such as proteins or lipids existing on fingertips adhere to the surfaces of the contact lenses in some cases in their handling. Adhesion of such stains causes a decrease in transparency of the contact lenses, leads to deterioration of wearing feeling such as the occurrence of a foreign-body sensation due to the stains adhered or deterioration of eyesight, and further, brings about ophthalmopathy such as corneitis or conjunctival hyperemia.

Consequently, in order to wear such contact lenses safely and comfortably, there have been proposed various contact lens cleaning agents for conducting a cleaning operation to the contact lenses when the contact lenses are removed from the eye in everyday handling. As a typical one thereof, a cleaning solution containing a degrading enzyme such as lipase or protease has been known (see patent documents 1 to 3). However, such a cleaning solution is effective to the cleaning operation to the contact lenses removed from the eye, but it must be essentially avoided that such a cleaning solution is used as an eye drop and applied to intend decomposition and removal of the stains for removing the stains on the contact lenses in a state where the contact lenses are worn on the eye, when an action of the degrading enzyme itself to ocular tissue is considered.

In order to further improve an effect of removing the stains such as eye lipids adhered to the contact lenses, it has also been considered that a specified surfactant is added to and contained in the contact lens solution (see patent document 4 and the like). With reference to the application of such a surfactant, it has been made clear in patent document 5 that an ophthalmic composition having good wettability to a contact lens is obtained by combining a specified nonionic surfactant and a viscous agent. Further, in patent document 6, an aqueous cleaning composition for a contact lens called AKYPO RLM-100 or the like has also been made clear which contains an anionic surfactant having a specific structure (a form in which a terminal -CH₂OH group of a polyoxyethylene long-chain alkyl ether is oxidized) as an active ingredient.

However, in a cleaning operation of the contact lens by a liquid composition containing such a surfactant, a high effect is obtained in an in vitro test for the effect of removing the lipid stain adhered to the contact lens, because of high lipid-solubilizing ability of the surfactant. However, when such a liquid composition is used as an eye drop or adhered to the contact lens to cause the surfactant to be introduced into the eye, there has been a fear of posing a problem that a normal tear fluid layer formed on the surface of eyeball is damaged to possibly cause damage to the healthy corneal epithelium.

For this reason, the surfactant-containing ophthalmic liquid composition has been a little insufficient in safety to living organisms. It has been therefore difficult to apply such an ophthalmic liquid composition as an eye drop in a state where the contact lens is worn on the eye, thereby wetting a surface of the contact lens, or further eliminating a dry feeling mainly recognized as an uncomfortable feeling during wear of the contact lens.

Patent Document 1: JP-A-7-76700
Patent Document 2: JP-A-4-370197
Patent Document 3: JP-A-9-87682
Patent Document 4: JP-A-10-108899
Patent Document 5: Pamphlet of International Publication No. 97/28827
Patent Document 6: U.S. Patent 4,808,239

### DISCLOSURE OF THE INVENTION

### PROBLEMS TO BE SOLVED BY THE INVENTION

The present invention has been made in view of such situations. It is therefore an object of the present invention to provide a solution composition for a contact lens high in safety to living organisms, which has a function of being able to remove stains such as a lipid stain adhered to the contact lens and a function of being able to effectively inhibit adhesion of stains such as a protein to a surface of the contact lens, and does not cause damage to the cornea due to damage of a tear fluid layer. Further, another object of the present invention is to provide an ophthalmic solution composition which can effectively wet a surface of a contact lens and can be suitably used as an eye drop for a contact lens.

### MEANS FOR SOLVING PROBLEMS

In order to achieve such objects, the present inventors have conducted extensive studies, and as a result, have found that an ophthalmic solution composition which does not affect a tear fluid layer or tear film while maintaining the function of solubilizing the lipid stain on the contact lens and the function of inhibiting adhesion of the protein stain to the contact lens can be obtained by allowing a specific nonionic surfactant having a track record of use as a compounding ingredient and said to be relatively safe to be contained at a specific low concentration, specifically in the vicinity of the critical micelle concentration, and allowing polyethylene glycol to be contained at a large ratio based on such a nonionic surfactant.

Accordingly, the present invention has been completed on the basis of such findings, and a first embodiment thereof is a solution composition for a contact lens, which comprises at least one nonionic surfactant selected from the group consisting of a polyoxyethylene sorbitan alkylate, polyoxyethylene hydrogenated caster oil, polyoxyethylene stearate and a polyoxyethylene-polyoxypropylene block copolymer in an aqueous medium within a range of 0.0001 to 0.04 w/w %, and comprises polyethylene glycol at a ratio of 20 parts by weight or more based on 1 part by weight of the nonionic surfactant.

In a second desirable embodiment of such a solution composition for a contact lens according to the present invention, the above-mentioned polyethylene glycol has an average molecular weight of 1,000 to 100,000.

Further, in a third embodiment of the solution composition for a contact lens according to the present invention, polyoxyethylene sorbitan monooleate is suitably used as the polyoxyethylene sorbitan alkylate.

Furthermore, in the present invention, a constitution that at least one thickener selected from the group consisting of hydroxyethyl cellulose, hydroxypropyl cellulose, hydroxypropylmethyl cellulose, polyvinylpyrrolidone, polyvinyl alcohol, chondroitin sulfate and hyaluronate is further contained in the solution composition for a contact lens is advantageously employed as a fourth desirable embodiment thereof.

In addition, according to a fifth preferred embodiment of the solution composition for a contact lens of the present invention, the osmotic pressure of the above-mentioned solution composition is adjusted to be within a range of 0.85 to 1.55 by the osmotic pressure ratio to physiological saline.

In the present invention, a constitution that at least one additive selected from the group consisting of an tonicity agent, a pH buffer, a chelating agent, a preservative, a germicide, a freshener, an antiphlogistic, a vasoconstrictor, a vitamin and an amino acid is further contained is also employed as a sixth embodiment.

Then, in the present invention, a seventh embodiment in which the solution composition as described above contains K⁺ within a range of 2 mEq/L to 30 mEq/L is advantageously employed.

Further, according to an eighth embodiment of the present invention, the solution composition as described above is advantageously used as an eye drop for a contact lens.

### EFFECT OF THE INVENTION

As described above, according to the above-mentioned first embodiment of the solution composition for a contact lens of the present invention, the specific nonionic surfactant said to be relatively safe as a compounding ingredient of an ophthalmic cleaning agent is contained at an extremely low concentration of 0.04 w/w % or less. Accordingly, even when such a nonionic surfactant exists, ability of solubilizing a lipid thereby is considered to be low. It is therefore conceivable that the surfactant does not have such force that the tear fluid layer is damaged to solubilize the whole oil film. For that reason, it does not happen to cause damage to the cornea due to damage of the tear fluid layer. On the other hand, although the concentration thereof is low, surface activity is imparted to the solution composition according to the present invention by the existence of the nonionic surfactant, thereby also exhibiting an effect of being able to effectively wet the surface of the contact lens.

Moreover, the present invention is also characterized by that even when the nonionic surfactant is contained at such a low concentration, the practically sufficient lipid stain-removing effect can be exhibited, and the lipid-solubilizing rate of the solution composition for a contact lens according to the present invention can be effectively improved by the existence of polyethylene glycol at a large ratio, thereby being able to enhance a function of removing stains such as a lipid stain. That is to say, in order to remove the lipid, it is necessary to first liquefy the lipid stain adhered to the surface of the contact lens in a cleaning process. Polyethylene glycol can permeate the lipid, which makes it possible to liquefy the stains and allow it to come up, thereby effectively bringing out action of the solubilizing function of the nonionic surfactant. This is considered to make it possible to exhibit the effective stain-removing effect.

Further, polyethylene glycol used in the present invention has a function of inducing precipitation thereof by interaction with a protein, thereby inhibiting or preventing the protein in tear fluid from being directly adhered to the surface of the contact lens, which also makes it possible to advantageously exhibit an effect of inhibiting adhesion of the stains such as the protein to the contact lens.

Accordingly, such a solution composition for a contact lens according to the present invention can effectively remove the stains such as the lipid stain adhered to the contact lens, also advantageously exhibit the effect of inhibiting adhesion of the stains such as the protein, and effectively wet the surface of the contact lens with its surface hydrophobilized. On the other hand, the solution composition does not cause problems such as damage to the cornea due to damage of the tear fluid layer and is excellent in safety. For this reason, the above-mentioned function and effect of the present invention can be advantageously exhibited by using it as the eye drop for a contact lens for application to the eye on which the contact lens is worn, as the above-mentioned eighth embodiment of the present invention.

The characteristic features of the present invention can be more advantageously exhibited by using one having an average molecular weight within the specified range as polyethylene glycol according to the second embodiment of the present invention, using polyoxyethylene sorbitan monooleate as the polyoxyethylene sorbitan alkylate according to the third embodiment of the present invention, and further adjusting the osmotic pressure of the solution composition within the specified range according to the fifth embodiment of the present invention.

Further, in the solution composition for a contact lens according to the present invention, the specified thickener is further contained according to the fourth embodiment of the present invention, or the specified additive is further contained according to the sixth embodiment of the present invention, thereby being able to impart a further characteristic feature to the solution composition for a contact lens, depending on each contained component.

In the present invention, the potassium ion (K⁺) concentration in the solution composition for a contact lens is adjusted so as to be within the specified range, as the above-mentioned seventh embodiment, which allows the solution composition to approach a tear fluid electrolyte composition, thereby being able to further improve biocompatibility of the solution composition. In general, in order to increase biocompatibility, an eye drop is adjusted in osmotic pressure to a degree similar to that of tear fluid, and sodium chloride (NaCl) is mainly used as a tonicity adjusting agent. However, although salt NaCl is a component indispensable to living organisms, it has been known that physiology is maintained by the existence of moderate amounts of other electrolytes such as K⁺ and Ca²⁺ at the same time. Of course, a moderate amount of potassium (K⁺) also exists in tear fluid, and it is desirable that potassium also exists in the eye drop in an amount nearly equal to that in tear fluid.

### BRIEF DESCRIPTION OF THE DRAWINGS

[Fig. 1] Fig. 1 is a graph showing the relationship between shaking time and measurement results of absorbance for respective test solutions and physiological saline obtained in Example 1.
[Fig. 2] Fig. 2 is a graph showing lipid-solubilizing rates for respective test solutions and physiological saline obtained in Example 1.
[Fig. 3] Fig. 3 is a graph showing the relationship between amounts of protein adhered to a contact lens and respective test solutions obtained in Example 3.

### BEST MODE FOR CARRYING OUT THE INVENTION

Now, such a solution composition for a contact lens according to the present invention is mainly composed of an aqueous medium, and contains a specified nonionic surfactant therein as one of essential ingredients at a low concentration. The nonionic surfactants used therein include a polyoxyethylene sorbitan alkylate, polyoxyethylene hydrogenated caster oil, polyoxyethylene stearate and a polyoxyethylene-polyoxypropylene block copolymer which have track records of use as a compounding ingredient of an ophthalmic solution and are said to be relatively safe, and at least one is selected therefrom to use. Of these, there is suitably used a polyoxyethylene sorbitan alkylate, particularly polyoxyethylene sorbitan monooleate, for example, various commercial products such as Polysorvate 80 (trade name: Nikko Chemicals Co., Ltd.), which is a component that can be advantageously enhanced in lipid-solubilizing ability, has many track records of use as a compounding ingredient of an ophthalmic solution in Japan, and shows mild surface activity.

Then, in order to achieve the objects of the present invention, such a nonionic surfactant is contained in the solution composition for a contact lens at a low concentration of 0.0001 to 0.04 w/w %, preferably 0.001 to 0.03 w/w %. When the content of such a nonionic surfactant is lower than the above-mentioned lower limit amount, it becomes far lower than the critical micelle concentration (CMC), which causes a problem of extremely lowered cleaning power. On the other hand, when the content is higher than the upper limit amount, there is posed a problem that the tear fluid layer is damaged to cause damage to the corneal epithelium.

Further, polyethylene glycol which is the other one of the essential ingredients of the solution composition for a contact lens according to the present invention is generally a polyether with hydroxyl groups at both terminal ends obtained by ring opening polymerization of ethylene oxide, and is used as a component for improving the lipid-solubilizing rate of the above-mentioned nonionic surfactant and inhibiting adhesion of the stains such as the protein stain. In order to sufficiently fulfill such functions, it is contained at a ratio of 20 parts by weight or more based on 1 part by weight of the nonionic surfactant. However, the content thereof in the solution composition for a contact lens is generally from 0.01 to 5.0 w/w %, and preferably from 0.1 to 1.0 w/w %. When the content of polyethylene glycol is too low, there is posed a problem that the composition becomes difficult to effectively function to solubilization of the lipid or a problem that the effect of preventing adhesion of the stains such as the protein stains becomes insufficient. On the other hand, when the content thereof becomes too high, a problem such as swelling or deformation of the contact lens becomes liable to occur.

As such polyethylene glycol, one having an average molecular weight ranging from 1,000 to 100,000 is preferably used, because adsorption and introduction into the contact lens are inhibited while sufficiently achieving the objects of the present invention, and sufficient solubility in water is concurrently secured. In particular, polyethylene glycol having a molecular weight ranging from 3,000 to 20,000 is advantageously used in the present invention among others.

Further, in the solution composition for a contact lens according to the present invention, at least one thickener for adjusting the viscosity of such a solution composition and enhancing moisture retention and wettability to impart a further excellent sense of use to the contact lens is further contained. Such thickeners include hydroxyethyl cellulose, hydroxypropyl cellulose, hydroxypropylmethyl cellulose, polyvinylpyrrolidone, polyvinyl alcohol, chondroitin sulfate and hyaluronate. Of these, hydroxypropylmethyl cellulose is advantageously used, because it has a track record of use as the compounding ingredient of the ophthalmic solution in Japan, and the effect of removing the lipid adhered to the contact lens is expected. The content of such a thickener is generally from about 0.05 to about 0.8 w/w%, and preferably from about 0.1 to about 0.8 w/w %. When the content of this thickener is low, there is a problem that a sufficient thickening effect is not obtained. On the other hand, exceeding the upper limit value results in an excessive increase in viscosity to cause a problem of a deteriorated sense of use or the like.

In addition, the solution composition for a contact lens according to the present invention may further contain one or two or more appropriately selected from the various additive components which have hitherto been used in the ophthalmic solution composition, at an usual content ratio, as well as the components as described above, without any hindrance as needed. It is preferred that such an additive component (additive) is high in safety to living organisms and ophthalmically allowable, and moreover has no influence on the shape and physical properties of the contact lens. Further, it is desirably used within the quantitative range satisfying such requirements. This makes it possible to advantageously impart various functions depending on the additive components to the solution composition according to the present invention without impairing the advantages of the present invention at all.

For example, in the solution composition for a contact lens according to the present invention, when the osmotic pressure thereof is too high or adversely too small, there is a fear of giving a stimulus to the eye, affecting the shape of the contact lens, or bringing about ophthalmopathy It is therefore desirable that the osmotic pressure of the solution composition for a contact lens is usually adjusted nearly to that of tear fluid by adding such as an tonicity agent (osmotic pressure regulating agent), and generally within a range of about 0.85 to about 1.55 and preferably within a range of about 0.9 to about 1.5, by the osmotic pressure ratio to physiological saline (osmotic pressure ratio of physiological saline taken as 1). As the osmotic pressure regulating agent used in such adjustment of the osmotic pressure, there is used at least one selected from the group consisting of sodium chloride, potassium chloride, a saccharide, a sugar alcohol, and a polyhydric alcohol, an ether thereof or an ester thereof.

Further, in the solution composition for a contact lens, when the pH value thereof is too high or adversely too small, there is a fear of giving a stimulus to the eye, or bringing about ophthalmopathy It is therefore desirable that the pH value of such a solution composition is usually adjusted to about 5.3 to about 8.5, especially to about 7, by adding an appropriate pH adjustor or buffer. As the pH adjustor used for such pH adjustment, there is used sodium hydroxide, hydrochloric acid or the like. On the other hand, as the pH buffer for keeping the pH of the solution composition effectively within the above-mentioned range and within a range safe to the eye, there is used one appropriately selected from conventionally known various ones. Specific examples thereof include acids such as phosphoric acid, boric acid, a carboxylic acid such as citric acid, and an oxycarboxylic acid, and salts thereof (such as a sodium salt), and further, Good-Buffer, tris (hydroxymethyl) aminomethane (TRIS), bis (2-hydroxyethyl) iminotris (hydroxymethyl) methane (Bis-Tris) and sodium hydrogen carbonate, because they are safe to the eye, and moreover, influences on the contact lens can be decreased.

In addition, there is a possibility that calcium or the like may be generally deposited or adsorbed on the contact lens, particularly on a soft contact lens, as stains from the tear. Accordingly, a chelating agent is also advantageously added to the solution composition for a contact lens, in order to prevent such deposition or adsorption of calcium or the like. Such chelating agents include, for example, ethylenediaminetetraacetic acid (EDTA), a salt thereof, such as disodium ethylenediaminetetraacetate (EDTA·2Na) or trisodium ethylenediaminetetraacetate (EDTA·3Na), and the like.

Further, in the solution composition for a contact lens according to the present invention, in order to advantageously exhibit a sterilization or germicidal effect to the eye and the contact lens and further a preservative or antiseptic effect of the solution composition for a contact lens, a preservative or germicide having preservative or germicidal activity is appropriately added to such a solution composition. As such preservatives or germicides, ones which are excellent in compatibility with the contact lens, as well as in preservative or germicidal activity, and are difficult to cause trouble such as allergy are generally desirable. They are appropriately selected from various known ones, and used alone or as a combination of a plurality of them.

The preservatives as used herein include, for example, sorbic acid, potassium sorbate, benzoic acid or a salt thereof, ethyl paraoxybenzoate, butyl parahydroxybenzoate, propyl parahydroxybenzoate, methyl parahydroxybenzoate, chlorobutanol, perboric acid or a perborate such as sodium perborate, chlorine dioxide and the like. On the other hand, the germicides include, for example, a biguanide-based germicide such as polyhexamethylene biguanide (PHMB) and a quaternary ammonium salt-based germicide such as polyquaternium. In particular, when such preservatives and germicides are not used, it is also possible to use as a single-dose type in which the ophthalmic composition according to the present invention is used up at a time or as a multi-dose type in which a discharging container provided with a filter as disclosed in JP-A-2002-80055 is used.

Furthermore, it is also possible to add a refrigerant such as menthol, borneol, camphor, geraniol, eucalyptus oil, bergamot oil, fennel oil, peppermint oil, rose oil or cool mint, for the purpose of presenting a refreshing sensation at the time of application of the eye drop to the eye or releasing an irritating or itching sensation when a contact lens is worn.

In addition, it is also possible to appropriately add various additional components such as an antiinflammatory such as glycyrrhizinic acid or a salt thereof, ε-aminocaproic acid, allantoin or sodium azulenesulfonate in order to control intraocular inflammation caused by stress or by wearing the contact lens, a vasoconstrictor such as naphazoline nitrate or tetrahydrozoline hydrochloride, which is said to act on the scleral vessels of the eye ball to eliminate congestion of the eye and to be effective for recovery from asthenopia, a vitamin such as vitamin A (including retinol palmitate and β-carotene), vitamin B₂, B₆ or B₁₂, vitamin E such as d-α-tocopherol acetate, or pantenol, asparaginic acid or a salt thereof, and an amino acid such as aminoethylsulfonic acid, arginine, alanine, lysine or glutamic acid, depending on the intended application of the solution composition for a contact lens.

Then, in the solution composition for a contact lens according to the present invention which comprises such various components, the concentration of potassium ions (K⁺) contained therein is adjusted generally within a range of about 2 mEq/L to about 30 mEq/L, and preferably within a range of about 4 mEq/L to about 28 mEq/L. When the potassium ion concentration is too high or too low, there is a fear of adversely affecting the ocular surface. The potassium ions are introduced from KCl added as the tonicity agent and from various additional components of the K salt type, and the total amount thereof in the solution composition is adjusted so as to be within the above-mentioned range.

By the way, the solution composition for a contact lens according to the present invention is prepared by adding and incorporating the above-mentioned components in respective proper amounts into an appropriate aqueous medium in a manner similar to the conventional one. It goes without saying that any one can be utilized as the aqueous medium used on that occasion, in addition to water itself such as city water, purified water or distilled water, as long as it is a solution mainly composed of water, high in safety to living organisms and ophthalmically sufficiently allowable. Further, in preparing such a solution composition, no particular method is required at all, and it can be easily obtained by dissolving the respective components in the aqueous medium in any order in the same manner as in the case where an ordinary aqueous solution is prepared.

The solution composition for a contact lens according to the present invention which is obtained as described above is excellent in safety to the eye, does not damage the tear fluid layer to cause damage to the corneal epithelium, and does not adversely affecting the shape or physical properties of the contact lens. Accordingly, it is advantageously used particularly as the eye drop, resulting in effectively exhibiting the above-mentioned function and effect of the present invention. When it is used as the eye drop, a proper amount thereof is applied to the eye in a state where the contact lens is worn on the eye, similarly to a conventionally known eye drop or an ophthalmic drug. Even for a contact lens with a lens surface hydrophobilized, the surface thereof is effectively wetted by the application to the eye at the time when the contact lens is worn, whereby an effect of reducing a dry sensation can be further maintained. Further, there is obtained the effect of removing the stains such as the fat stain adhered to the lens surface or advantageously inhibiting adhesion of the stains such as the protein.

Further, when the solution composition for a contact lens according to the present invention is used as a solution for a contact lens, not as the eye drop, for example, the contact lens is cleaned or rinsed with such an ophthalmic composition, and then, the contact lens is worn in a state where the solution composition is adhered onto a surface of the contact lens, or the contact lens is immersed and kept in such a solution composition for a definite period of time, and then, the contact lens is taken out and may be worn as it is. When the composition is used like this, the effect by the solution composition for a contact lens according to the present invention is advantageously exhibited.

The above-mentioned solutions for a contact lens include all of the known solutions which have hitherto been used for care of the contact lens, such as a multi-purpose solution intending two or more applications of cleaning, rinsing, germiciding and preservation, as well as a cleaning solution for a contact lens, a rinsing solution for a contact lens and a solution for preserving a contact lens which intend a single application, and its application is not limited at all.

Further, when the solution composition for a contact lens according to the present invention is used as the eye drop for a contact lens or as the solution for a contact lens, the type of contact lens to which the solution composition is applied is not limited at all, and the solution composition can be applied to hard contact lenses, and soft contact lenses which may be classified into, for example, all of non-water-content, low-water-content and high-water-content contact lenses. The material and the like of the contact lens are not called into question at all when the present invention is applied.

### EXAMPLES

The present invention will be described in further detail with reference to typical examples of the present invention, but it goes without saying that the present invention is by no means restricted by a description of such examples. In addition to the following examples and further the above-mentioned specific descriptions, it should be understood that various changes, modifications and improvements may be made to the present invention, based on knowledge of those skilled in the art without departing from the gist of the present invention.

### -Example 1-

First, various test solutions 1 to 7 adjusted in pH to 6.5 were each prepared by adding prescribed compounding components at respective ratios shown in the following Table 1 to purified water. All the resulting test solutions of 7 kinds showed an osmotic pressure of about 1.1, by the osmotic pressure ratio to physiological saline.

In addition, HPMC2910 used in the preparation of such test solutions is hydroxypropylmethyl cellulose (METOLOSE TC-5E manufactured by Shin-Etsu Chemical Co., Ltd.) as the thickener, and PEG#6000 is polyethylene glycol having an average molecular weight of 6000 (manufactured by Fluka Bio Chemika). Further, Polysorbate 80 is polyoxyethylene sorbitan monooleate (TO-10M manufactured by Nikko Chemicals Co., Ltd) as the nonionic surfactant. The amount of borax used was determined at such a ratio that the pH of each test solution was adjusted to 6.5.

**[TABLE 1]**

| | Compounding Component | Test Solution 1 | Test Solution 2 | Test Solution 3 | Test Solution 4 | Test Solution 5 | Test Solution 6 | Test Solution 7 |
|---|---|---|---|---|---|---|---|---|
| Compound ing Ratio [W/W %] | HPMC2910 | 0.65 | 0.65 | 0.65 | 0.65 | 0.65 | 0.65 | 0.65 |
| | Boric Acid | 1.0 | 1.0 | 1.0 | 1.0 | 1.0 | 1.0 | 1.0 |
| | Borax | Proper amount | Proper amount | Proper amount | Proper amount | Proper amount | Proper amount | Proper amount |
| | Sodium Edetate | 0.011 | 0.011 | 0.011 | 0.011 | 0.011 | 0.011 | 0.011 |
| | Sodium Chloride | 0.65 | 0.65 | 0.65 | 0.65 | 0.65 | 0.65 | 0.65 |
| | Potassium Chloride | 0.10 | 0.10 | 0.10 | 0.10 | 0.10 | 0.10 | 0.10 |
| | PEG#6000 | 0.50 | - | 0.50 | - | 0.50 | 0.20 | 0.10 |
| | Polysorvate 80 | 0.010 | 0.010 | 0.10 | 0.10 | - | 0.010 | 0.010 |
| | Purified Water | q.s.100 | q.s.100 | q.s.100 | q.s.100 | q.s.100 | q.s.100 | q.s.100 |

| | | | | | | | | |
|---|---|---|---|---|---|---|---|---|
| q.s.100: quantum sufficit 100 | | | | | | | | |

Then, using such prepared test solutions 1 to 5 and physiological saline, the cleaning effect of those solutions to a lipid was examined by a lipid-solubilizing rate method.

Specifically, using a colored lipid obtained by mixing triglyceride as a lipid and Sudan I as a pigment at a weight ratio of 99:1, 0.5 g thereof was accommodated in a specified test bottle, and then, 20 mL of the test solution obtained above or physiological saline was further added and accommodated in the test bottle. Thereafter, an operation of covering an opening of the test bottle with a proper lid was carried out for each solution sample. Then, each test bottle thus prepared in which the colored lipid and the solution sample were accommodated was shaken at a constant rate at a temperature of 25°C for 1 to 24 hours, and further allowed to stand for a predetermined time. Thereafter, a supernatant fluid in each test bottle was collected, and the absorbance at 485.5 nm was measured for each of those supernatant fluids with a spectrophotometer (self-recording spectrophotometer UV-2200 manufactured by Shimadzu Corporation).

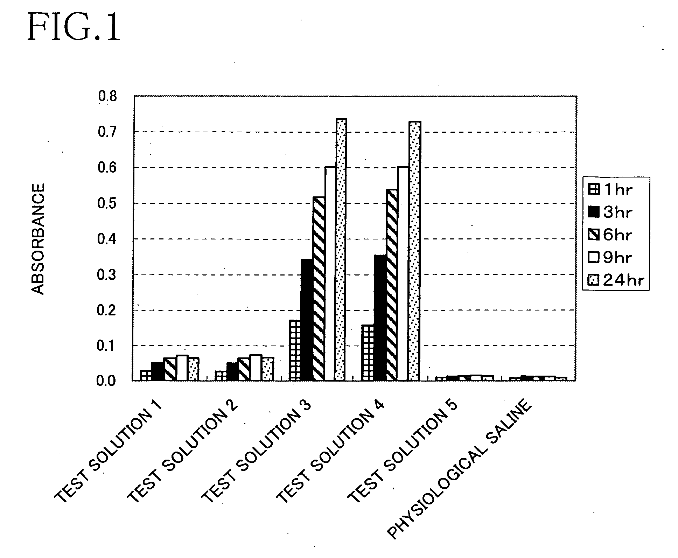

Then, the absorbance of the supernatant fluid in each test bottle was measured at the times when 1 hour, 3 hours, 6 hours, 9 hours and 24 hours had elapsed as the shaking time to the above-mentioned test bottle. The results of measurement are shown in the following Table 2, and also in Fig. 1.

**[TABLE 2]**

| | | Shaking Time | | | | |
|---|---|---|---|---|---|---|
| | Kind of Solution | 1 hr | 3 hr | 6 hr | 9 hr | 24 hr |
| | Test Solution 1 | 0.028 | 0.050 | 0.064 | 0.072 | 0.065 |
| Absorbance | Test Solution 2 | 0.027 | 0.050 | 0.064 | 0.073 | 0.066 |
| | Test Solution 3 | 0.171 | 0.342 | 0.517 | 0.602 | 0.737 |
| | Test Solution 4 | 0.158 | 0.354 | 0.538 | 0.602 | 0.729 |
| | Test Solution 5 | 0.010 | 0.013 | 0.014 | 0.015 | 0.014 |
| | Physiological Saline | 0.008 | 0.013 | 0.012 | 0.012 | 0.009 |

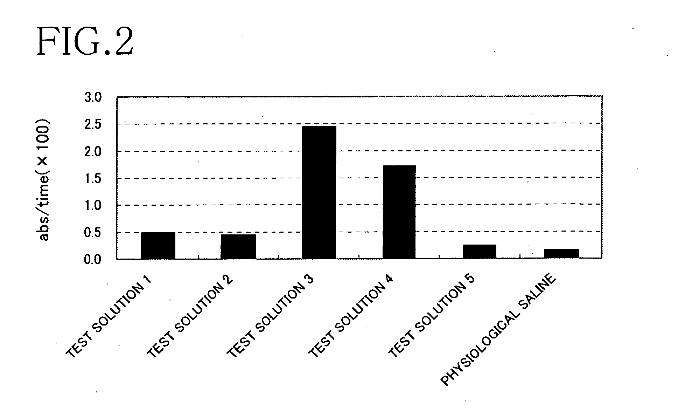

Thereafter, for the respective test solutions and physiological saline, the lipid-solubilizing rate was determined as an intercept gradient of the absorbance until after 3 hours measured above. The results thereof are shown in the following Table 3, and also in Fig. 2 in the form of a bar graph.

**[TABLE 3]**

| Kind of Solution | abs/time (×100) |
|---|---|
| Test Solution 1 | 0.486 |
| Test Solution 2 | 0.443 |
| Test Solution 3 | 2.443 |
| Test Solution 4 | 1.714 |
| Test Solution 5 | 0.243 |
| Physiological Saline | 0.157 |

As apparent from the results of such a lipid-solubilizing test, it is recognized that test solution 1 is higher than test solution 2 and test solution 3 is higher than test solution 4, in the value of the lipid-solubilizing rate, although test solution 1 and test solution 2, and test solution 3 and test solution 4 show an approximately similar value in the lipid-solubilizing amount. Then, inspecting the difference in composition among the respective test solutions, it is understood that polyethylene glycol and the nonionic surfactant (Polysorvate 80) are used together in both test solution 1 and test solution 3, whereas test solution 2 and test solution 4 contain only the nonionic surfactant (Polysorvate 80) of these two substances (contain no polyethylene glycol).

With the above in mind, it is understood that polyethylene glycol increases only the lipid-solubilizing rate without decreasing the lipid-solubilizing amount due to surface activity of the nonionic surfactant by using in combination with the nonionic surfactant. Further, giving no change to the lipid-solubilizing amount and increasing only the lipid-solubilizing rate results in more rapidly working on a lipid stain component such as eye lipids in applying the solution to the eye to be able to advantageously conduct its removing treatment. Accordingly, a cleaning solution effective in a cleaning function can be obtained by using polyethylene glycol together with the nonionic surfactant, rather than by using the nonionic surfactant alone.

### -Example 2-

Based on the results of Example 1 described above, in order to weigh test solution 1 and test solution 3 in which the two components of polyethylene glycol and the nonionic surfactant (Polysorvate 80) were used in combination, the Ni-BUT (Non-invasive Tear Break-up Time) measurement test for examining the influence on a surface of the eye was performed.

Specifically, the above-mentioned test solution 1 was applied to the eye in a state in which a commercially available contact lens (ACUVUE2 manufactured by Johnson & Johnson K.K.) was worn on the eye of a single subject, and Ni-BUT of tear fluid on a front surface of the contact lens was measured for every one minute. As a measurement instrument, there was used a dry eye observing device DR-1 manufactured by Kowa Company, Ltd. Further, after the Ni-BUT measurement test for such test solution 1 it was confirmed that the tear fluid returned to a normal state. Thereafter, test solution 3 prepared in Example 1 was further applied to the eye, and Ni-BUT of tear fluid on the front surface of the contact lens was similarly measured for every one minute. Then, after an elapse of 30 minutes after the application thereof to the eye, the measurement was made at intervals of 5 minutes. The measurement results thereof are shown in The following Table 4.

**[TABLE 4]**

| Elapsed Time after Application to Eye [min] | 1 | 2 | 3 | 4 | 5 | 6 | 7 | 8 | 9 | 10 | 11 |
|---|---|---|---|---|---|---|---|---|---|---|---|
| Test Solution 1 [sec] | 10 | 7 | 5 | 5 | 5 | 5 | 5 | 6 | 8 | 9 | 10 |
| Test Solution 3 [sec] | 10 | 8 | 8 | 5 | 6 | 5 | 4 | 4 | 4 | 3 | 3 |
| | | | | | | | | | | | |

| Elapsed Time after Application to Eye [min] | 12 | 13 | 14 | 15 | 16 | 17 | 18 | 19 | 20 | 21 | 22 |
|---|---|---|---|---|---|---|---|---|---|---|---|
| Test Solution 1 [see] | - | - | - | - | - | - | - | - | - | - | - |
| Test Solution 3 [see] | 5 | 4 | 4 | 3 | 4 | 7 | 7 | 5 | 5 | 6 | 3 |
| | | | | | | | | | | | |

| Elapsed Time after Application to Eye [min] | 23 | 24 | 25 | 26 | 27 | 28 | 29 | 30 | 35 | 40 | 45 |
|---|---|---|---|---|---|---|---|---|---|---|---|
| Test Solution 1 [see] | - | - | - | - | - | - | - | - | - | - | - |
| Test Solution 3 [sec] | 3 | 4 | 4 | 3 | 7 | 6 | 5 | 5 | 5 | 6 | 10 |

As apparent from such results of the Ni-BUT measurement test shown in Table 4, it takes about 10 minutes to return to "Ni-BUT=10 seconds", which is a value before the application to the eye of the subject, in test solution 1 in which the Polysorvate 80 as the nonionic surfactant is formulated at a concentration in the vicinity of the critical micelle concentration. In contrast, it takes a time as long as 45 minutes to return to "Ni-BUT=10 seconds" in test solution 3 in which Polysorvate 80 is formulated at a concentration about 10 times the critical micelle concentration. From this, it has been confirmed that a tear fluid layer is more damaged, as the concentration of Polysorvate 80 as the nonionic surfactant becomes higher, which prolongs the time taken to return to normal tear fluid. Further, from this, it is understood that test solution 1 in which the concentration of Polysorvate 80 as the nonionic surfactant is set to a concentration in the vicinity of the critical micelle concentration becomes very safe to the eye and is preferred, rather than test solution 3 in which it is set to a concentration about 10 times the critical micelle concentration.

### -Example 3-

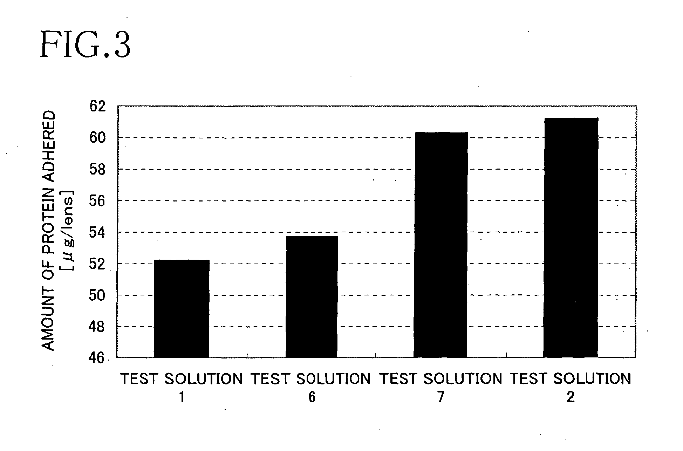

Using test solutions 1, 2, 6 and 7 prepared in Example 1, those test samples were further adjusted to pH=7.3 with a proper amount of borax, and then, 2 mL of each solution was accommodated in a vial bottle. Thereafter, three commercially available contact lenses ("1· DAY ACUVUE" manufactured by Johnson & Johnson K.K.) were each immersed in each vial bottle for 2 days. Then, the contact lenses thus immersion treated were accommodated one by one in a vial bottle (kept at 25°C) containing 5 mL of artificial tear fluid (lysozyme: 496 µg/mL, sodium chloride: 0.9%, calcium chloride: 1 mM, sodium dihydrogen phosphate: 4 mM, pH=7.0), and immersed therein. After the immersion for 5 minutes, the concentration of lysozyme in the artificial tear liquid was measured by high performance liquid chromatography. The amount of lysozyme adhered to the contact lenses was calculated from the difference between the concentration of lysozyme in the artificial tear liquid before the immersion of the contact lenses and the concentration of lysozyme in the artificial tear liquid after the immersion of the contact lenses. An average value of three measurements (n=3) was determined. The results thereof are shown in the following Table 5. Further, in order to weigh them, the results of Table 5 are shown in the order of test solution 1, test solution 6, test solution 7 and test solution 2 in Fig. 3 by a bar graph. The amount of lysozyme adhered to the lenses treated with physiological saline for comparison was 96.0 µg/lens.

**[TABLE 5]**

| | Test Solution 1 | Test Solution 2 | Test Solution 6 | Test Solution 7 |
|---|---|---|---|---|
| Amount of Lysozyme Adhered [µg/lens] | 52.2 | 61.2 | 53.7 | 60.3 |

As apparent from such results of the table, there is a large difference between test solution 6 containing 0.01% of the nonionic surfactant (Polysorvate 80) and 0.10% of polyethylene glycol and test solution 7 containing 0.01% of the nonionic surfactant (Polysorvate 80) and 0.20% of polyethylene glycol, and a significant difference is observed therein between 1:10 and 1:20 of the ratio of Polysorvate 80 and polyethylene glycol. It can be found that the former test solution 6 is smaller in the amount of protein adhered than the latter test solution 7. In addition, considering that the viscosity excessively increases when high-molecular weight polyethylene glycol is contained at a high concentration, it is considered that the amount ratio of the nonionic surfactant (Polysorvate 80) and polyethylene glycol is preferably within a range of 1:20 to 1:50.

### -Example 4-

In a test solution prepared in the same manner as in Example 1 using polyoxyethylene hydrogenated caster oil, polyoxyethylene stearate or a polyoxyethylene-polyoxypropylene block copolymer (manufactured by BASF) as another nonionic surfactant in place of Polysorvate 80 as the nonionic surfactant used for the preparation of the test solution in Example 1, and in the case in which one having an average molecular weight of 4000 (manufactured by NACALAI TESQUE, Inc.) was used as polyethylene glycol, there could also be obtained an ophthalmic solution composition which could effectively remove lipid stains adhered to surfaces of contact lenses worn on the eye, effectively inhibited adhesion of proteins and was safe to living organisms.

### -Example 5-

A similar test solution was prepared, changing the compounding ratio of Polysorvate 80 as the nonionic surfactant, which is a compounding component of test solution 1 in Example 1, to 0.001 w/w %, and changing the amount of polyethylene glycol (PEG#6000) incorporated to 0.05 w/w %. As a result, it was observed that the resulting test solution was inferior in the absolute value of its lipid-solubilizing amount to test solution 1 in Example 1, but indicated a higher value than physiological saline. Further, in the BUT measurement test, even when it was applied to the eye similarly to the case of Example 2, the time taken for tear fluid to return to a normal state was fast, because of the small content of the nonionic surfactant (Polysorvate 80). It was therefore observed to be a much safer ophthalmic solution composition.

## Claims

1. A solution composition for a contact lens, which comprises at least one nonionic surfactant selected from the group consisting of a polyoxyethylene sorbitan alkylate, polyoxyethylene hydrogenated caster oil, polyoxyethylene stearate and a polyoxyethylene-polyoxypropylene block copolymer in an aqueous medium within a range of 0.0001 to 0.04 w/w %, and comprises polyethylene glycol at a ratio of 20 parts by weight or more based on 1 part by weight of said nonionic surfactant.

2. A solution composition for a contact lens according to claim 1, wherein said polyethylene glycol has an average molecular weight of 1,000 to 100,000.

3. A solution composition for a contact lens according to claim 1 or 2, wherein said polyoxyethylene sorbitan alkylate is polyoxyethylene sorbitan monooleate.

4. A solution composition for a contact lens according to any one of claims 1 to 3, wherein the composition further comprises at least one thickener selected from the group consisting of hydroxyethyl cellulose, hydroxypropyl cellulose, hydroxypropylmethyl cellulose, polyvinylpyrrolidone, polyvinyl alcohol, chondroitin sulfate and hyaluronate.

5. A solution composition for a contact lens according to any one of claims 1 to 4, wherein the osmotic pressure of said solution composition is within a range of 0.85 to 1.55 by the osmotic pressure ratio to physiological saline.

6. A solution composition for a contact lens according to any one of claims 1 to 5, wherein the composition further comprises at least one additive selected from the group consisting of an tonicity agent, a pH buffer, a chelating agent, a preservative, a germicide, a freshener, an antiphlogistic, a vasoconstrictor, a vitamin and an amino acid.

7. A solution composition for a contact lens according to any one of claims 1 to 6, wherein said solution composition contains K⁺ within a range of 2 mEq/L to 30 mEq/L.

8. A solution composition for a contact lens according to any one of claims 1 to 7, wherein the composition is used as an eye drop for a contact lens.
